# FASCICULE DE BREVET EUROPEEN

(11) **EP 1 404 817 B1**
(45) Date de publication et mention de la délivrance du brevet: **22.03.2006**
(21) Numéro de dépôt: 02784858.9
(22) Date de dépôt: 08.07.2002
(51) Int. Cl.: C12N 5/08, A61K 39/00, A61P 31/00, A61P 35/00

(54) **UTILISATION DE LIPOPROTEINES OXYDEES POUR OBTENIR LA DIFFERENCIATION DE CELLULES PRECURSEUR EN CELLULES DENDRITIQUES MATURES**
VERWENDUNG OXIDIERTER LIPOPROTEINE ZUR DIFFERENZIERUNG VON VORLÄUFERZELLEN IN REIFE DENDRITISCHE ZELLEN
USE OF OXIDISED LIPOPROTEINS FOR DIFFERENTIATION OF PRECURSOR CELLS INTO MATURE DENDRITIC CELLS

(30) Priorité: 09.07.2001 FR 0109103
(43) Date de publication de la demande: 07.04.2004
(73) Titulaire: BIOMERIEUX, 69280 Marcy-L'Etoile (FR); INSTITUT NATIONAL DE LA SANTE ET DE LA RECHERCHE MEDICALE (INSERM), 75654 Paris Cédex 13 (FR)
(72) Inventeur: LOTTEAU, Vincent, F-69390 Vourles (FR); ANDRE, Patrice, F-69003 Lyon (FR)
(74) Mandataire: Guerre, Dominique
(86) Numéro de dépôt international: PCT/FR2002/002390
(87) Numéro de publication internationale: WO 2003/006634

(56) Documents cités:
- WO-A-01/24818
- WO-A-97/29182
- HOFER E ET AL: "The centromeric part of the human natural killer (NK) receptor complex: lectin-like receptor genes expressed in NK, dendritic and endothelial cells." IMMUNOLOGICAL REVIEWS, vol. 181, juin 2001 (2001-06), pages 5-19, XP002196475 ISSN: 0105-2896
- PALINSKI W ET AL: "Low density lipoprotein undergoes oxidative modification in vivo" PROCEEDINGS OF THE NATIONAL ACADEMY OF SCIENCES OF USA, vol. 86, 1 février 1989 (1989-02-01), pages 1372-1376, XP002095482 ISSN: 0027-8424
- PERRIN-COCON L ET AL: "Oxidized low-density lipoprotein promotes mature dendritic cell transition from differentiating monocyte." JOURNAL OF IMMUNOLOGY, vol. 167, no. 7, 1 octobre 2001 (2001-10-01), pages 3785-3791, XP002196476 ISSN: 0022-1767
- COUTANT F ET AL: "Mature dendritic cell generation promoted by lysophosphatidylcholine." JOURNAL OF IMMUNOLOGY, vol. 169, no. 4, 15 août 2002 (2002-08-15), pages 1688-1695, XP002224601 ISSN: 0022-1767
- SOBANOV Y ET AL: "A novel cluster of lectin-like receptor genes expressed in monocytic, dendritic and endothelial cells maps close to the NK receptor genes in the human NK gene complex." EUROPEAN JOURNAL OF IMMUNOLOGY, vol. 31, no. 12, décembre 2001 (2001-12), pages 3493-3503, XP002224602 ISSN: 0014-2980
- ALDERMAN C J J ET AL: "Effects of oxidised low density lipoprotein on dendritic cells: A possible immunoregulatory component of the atherogenic micro-environment?" CARDIOVASCULAR RESEARCH, vol. 55, no. 4, septembre 2002 (2002-09), pages 806-819, XP002224603 ISSN: 0008-6363

## Description

La présente invention concerne l'utilisation d'au moins une fraction de lipoprotéines oxydées choisies parmi les VLDL oxydées et/ou les IDL oxydées et/ou LDL oxydées pour obtenir la différenciation de cellules précurseur, notamment de monocytes en cellules dendritriques matures.

Les cellules dendritiques sont impliquées dans le développement d'une réponse immune et dans l'initiation d'une réponse lymphocytaire T spécifique par reconnaissance, capture et présentation des antigènes notamment des agents infectieux (Steinman et al. 1997, Immuno. Rev., 156: 25-37 ; Cella et al. 1997, Curr. Opin. Immunol., 9 : 10-16).

Les cellules dendritiques immatures sont localisés dans les tissus non lymphoïdes. Ces cellules sont également appelées cellules de Langerhans lorsqu'elles sont localisées au niveau de la peau, et dans tous les épidermes hormis l'intestin. Des cellules dendritiques, en plus faible quantité, sont également localisées dans les organes tels que le foie, le poumon, l'intestin. Ces cellules dendritiques immatures captent et digèrent les antigènes des agents infectieux de façon très efficace.

Si les cellules dendritiques existent à l'état immature dans le plupart des tissus, certains signaux, comme les agents bactériens ou les cytokines inflammatoires, peuvent les activer en induisant un processus de maturation. L'activation des cellules dendritiques est l'étape initiale du déclenchement de la réponse immunitaire adaptative. En effet, au cours de cette activation, les cellules dendritiques acquièrent la capacité à migrer vers les organes lymphoïdes où se trouvent les lymphocytes T et la capacité à transmettre des signaux de co-stimulation indispensables à l'activation des lymphocytes T naïfs. Ainsi, durant la migration des cellules dendritiques vers les organes lymphoïdes, les cellules dendritiques subissent des modifications fonctionnelles et phénotypiques que l'on regroupe sous le terme de maturation, qui se caractérise par :
1) une augmentation à la surface des cellules dendritiques de molécules impliquées dans l'activation des lymphocytes T (telles que CD40, CD80, CD83, CD86 et les molécules du complexe majeur d'histocompatibilité (CMH) de classe I et II)
2) la production de cytokines proinflammatoires (telles que les interleukines IL-12, IL-1β et IL-6).
3) la diminution de la capacité des cellules dendritiques à capter et à digérer l'antigène.

De part leurs capacités de développement d'une réponse immune et d'initiation d'une réponse lymphocytaire T spécifique, les cellules dendritiques présentent un intérêt thérapeutique particulier notamment dans les domaines de vaccination anti-infectieuse et anti-tumorale et de l'immunothérapie (Austin. 1998, Curr. Opin. Hematol. 5 : 3-15 ; Reise Sousa et al. 1999, Curr. Opin. Immunol. 11 : 392-399).

*In vivo,* les monocytes sont des cellules circulantes qui peuvent pénétrer dans les tissus. En traversant la paroi vasculaire endothéliale les monocytes entrent au contact de facteurs environnementaux qui influencent leur devenir. Schématiquement, on envisage trois possibiblités pour ces cellules 1) la sortie des tissus et le retour vers les ganglions lymphatiques, 2) la différenciation en macrophages, 3) la différenciation en cellules dendritiques immatures. La nature des facteurs environnementaux endogènes qui orientent les monocytes vers l'une ou l'autre de ces voies est encore méconnue.

*In vitro,* la première étape pour obtenir des cellules dendritiques matures à partir de monocytes est une induction de la différenciation des monocytes en culture en cellules dendritiques immatures par notamment l'interleukine IL-4 et le facteur GM-CSF (Granulocyte Macrophage-Stimulating factor). Au bout de 6 jours, 95% des cellules en culture sont des cellules dendritiques immatures. La deuxième étape est une activation de la maturation des cellules dendritiques immatures qui est généralement induite par des agents exogènes, tels que des agents bactériens ou viraux ou tout autre molécules susceptibles d'induire la maturation. Il a ainsi été décrit par Pascale Jeannin et al. (Nature Immunology, 2000, 1 : 502-509) que la protéine kpOmpA de *Klebsiella pneumoniae,* ajoutée au 6^{e} jour de culture sur des cellules dentritiques immatures était capable d'induire la maturation de ces cellules. Comme variante de la deuxième étape, on peut citer la demande WO-A-97/29182 qui décrit l'ajout dans le milieu de culture, durant 3 jours, d'un milieu conditionné comprenant un facteur de maturation des cellules dentritiques, tels que les gamma-globuline ou un agent bactérien de Staphylococcus aureus (pansorbine) pour obtenir la maturation des cellules dendritiques.

On s'aperçoit ainsi que le délai pour l'obtention de cellules dendritiques matures est substantiellement long puisqu'il nécessite au moins 6 jours pour arriver à l'état de cellules dendritiques immatures et au moins 2 jours pour leur maturation.

Par ailleurs, l'utilisation de molécules exogènes dérivés d'agents infectieux pour initier la maturation des cellules dendritiques est difficilement envisageable dans le cadre de la vaccinologie et de l'immunothérapie, de part les problèmes sécuritaires et financiers liés à l'utilisation d'agents infectieux (effets secondaires directs ou indirects *in vivo ;* nécessité de purification très importante selon des exigences légales ou réglementaires très strictes).

L'invention se propose de répondre à l'ensemble des inconvénients de l'état de la technique, en diminuant d'une part le temps nécessaire à l'obtention de cellules dendritiques matures, et en évitant d'autre part l'utilisation de molécules exogènes dérivés d'agents infectieux pour activer la maturation.

D'une façon surprenante, les inventeurs ont mis en évidence que les lipoprotéines oxydés, notamment des LDL oxydés (Low Density Lipoprotein oxydées), permettent la différenciation de cellules précurseur, telles que notamment les monocytes en culture en cellules présentant les caractéristiques morphologiques et phénotypiques de cellules dendritiques matures. Les lipoprotéines sont des particules sphériques solubles dans l'eau qui transportent des lipides non polaires. Chez l'homme, les LDL sont les principaux transporteurs du cholestérol, et sont formés d'un coeur hydrophobe contenant des molécules d'ester de cholesterol et d'une enveloppe formée d'une couche de lipides polaires (principalement des phospholipides) dans laquelle s'insère l'apolipoprotéine B. Les LDL plasmatiques peuvent traverser l'endothélium et subir des modifications oxydatives notamment dans l'espace subendothélial (Witztum et al. 1991, J. Clin. Invest., 88 : 1785-1792 ; Steinberg et al. 1988, In *atherosclerosis reviews*. Raven Press, NY, 1-23). Les LDL oxydés présentent des activités inflammatoires. Les modifications oxydatives pro-inflammatoires des LDL sont sous le contrôle des LDL natives et des HDL natives (High Density Lipoprotein). Il a été montré notamment que les enymes paraoxonase et «platelet-activating factor acetylhydrolase» (ou PAF-AH) préviennent, dans les conditions normales l'accumulation de LDL oxydés (Mackness et al. 1991, FEBS Lett., 286 :152-154 ; Watson et al. 1995, J. Clin. Invest. 96 : 2882-2891; Watson et al., 1995, J. Clin. Invest. 95 : 774-782). Lors de la phase aiguë d'une agression, des modifications importantes apparaissent dans le plasma et en particulier dans la composition des lipoprotéines. Par exemple, la composition des HDL varie, ce qui a pour conséquence de transformer des HDL anti-inflammatoires en HDL pro-inflammatoires. Les LDL oxydés stimulent notamment l'expression de MCP-1 (Monocyte Chemoattractant Protein-1), M-CSF (Macrophage - Colony Stimulating Factor) et GM-CSF (Granulocyte Macrophage - Colony stimulating factor) par les cellules endothéliales et augmentent l'adhérance des monocytes et leur transmigration à travers les cellules endothéliales (Kruth, 1985, Atherosclerosis, 57 : 337-341 ; Simionescu et al, 1986, Am. J. Pathol., 123 : 109-125 ; Frank et al. 1989, J. Lipid Res., 30 : 967-978 ; Rajavashisth et al. 1990, Nature, 344 : 254-257 ; Navab et al. 1991, J. Clin. Invest., 88 : 2039-2046).

A ce jour, aucun élément de l'art antérieur ne décrit, ni ne suggère la différenciation de cellules précurseur telle que notamment des monocytes en cellules dendritiques matures en présence de LDL oxydés.

Ainsi, la présente invention concerne l'utilisation d'au moins une fraction de lipoprotéines oxydées choisies parmi les VLDL (Very Low Density Lipoprotein) oxydées et/ou les IDL (Intermediate Density Lipoprotein) oxydées et/ou LDL (Low Density Lipoprotein) oxydées pour obtenir la différenciation de cellules précurseur en cellules dendritriques matures. Par cellules précurseur, on entend des cellules capables de se différencier en cellules dendritiques, telle que notamment les monocytes ou les cellules CD34+, isolées notamment à partir de sang de cordon ou de moelle osseuse. Par cellules précurseur, on entend également des cellules pluripotentes ou des cellules immatures ayant la capacité de se différencier en cellules dendritiques immatures et matures. Il peut s'agir de cellules isolées et purifiées ou de lignées cellulaires cultivées *in vitro.* Dans un mode de réalisation de l'invention les cellules précurseur se différencient en cellules dendritiques immatures en présence d'IL-4 et de GM-CSF. Selon un mode préféré de l'invention, les cellules précurseur sont des monocytes. Dans un mode de réalisation préféré de l'invention, les monocytes sont isolés de sang périphérique humain. Selon d'autres modes de réalisation les monocytes sont isolés de souris, de rats, de lapins, de singes ou de tout autre mammifère.

Selon un mode préféré de l'invention, la différenciation des monocytes en cellules dendritiques matures est réalisée *in vitro*.

Les fractions sont obtenues à partir de centrifugation de plasma selon un protocole classique connu de l'homme du métier et définies par leur densité : densité inférieure à 1,0063 g/ml pour la fraction VLDL, densité comprise entre 1,025 et 1,0063 g/ml pour les IDL et densité comprise entre environ 1,06 et 1,025 g/ml pour les LDL, de préférence entre 1,055 et 1,025 g/ml.

La présente invention concerne aussi l'utilisation d'au moins une fraction de lipoprotéines oxydées choisie parmi les VLDL oxydées et/ou les IDL oxydées et/ou LDL oxydées pour la fabrication d'un médicament destiné à obtenir la différenciation de cellules précurseur, notamment de monocytes en cellules dendritiques matures.

Selon un mode préféré de l'invention, ce médicament peut servir à la production de cellules dendritiques matures après mise en contact avec un agent biologique. On obtient ainsi grâce à ce médicament des cellules dendritiques matures présentant un antigène donné, ce qui induit et/ou augmente la réponse immunitaire du patient recevant ledit médicament.

Cet agent biologique peut être notamment choisi parmi les antigènes de bactéries, de virus, de levures, de parasites, de champignons, les antigènes tumoraux, les lysats de cellules tumorales autologues et/ou hétérologues. Cet agent biologique peut être également un acide nucléique qui code au moins un antigène choisi parmi les antigènes de bactéries, de virus, de levures, de parasites, de champignons, les antigènes tumoraux.

Au sens de la présente invention, un antigène tumoral est défini comme une protéine ou un peptide tumoral, et notamment comme un épitope, notamment un épitope CTL (lymphocyte T cytotoxique) (séquences peptidiques interagissant avec les molécules de classe I et présentés aux lymphocytes T CD8+) ou comme la séquence nucléique codant pour cet antigène. On peut citer à titre non limitatif les antigènes tumoraux suivants : MAGE-2, MAGE-3, MART, MUC-1, MUC-2, HER-2, GD2, carcinoembryonic antigen (CEA), TAG-72, ovarian-associated antigens OV-TL3 et MOV18, TUAN, alpha-feto protein (AFP), OFP, CA-125, CA-50, CA-19-9, renal tumor-associated antigen G250, EGP-40 (ou EpCAM), S100 (maligmant meanoma-associated antigen), p53, prostate tumor-associated antigens (e.g., PSA et PSMA) et p21ras.

Au sens de la présente invention, on entend par cellules tumorales autologues, des cellules de tumeurs appartenant au sujet qui va recevoir le médicament selon l'invention. Par cellules tumorales hétérologues, on entend des cellules issues de tumeurs provenant d'un sujet différent de celui qui va recevoir le médicament selon l'invention. L'utilisation de cellules tumorales hétérologues permet notamment d'obtenir un médicament permettant de traiter des patients atteints de cancer chez qui un prélèvement de cellules tumorales n'est pas possible. Les cellules tumorales peuvent être obtenues par prélèvement de tissus cancéreux, notamment une biopsie ou une résection chirurgicale.

Au sens de la présente invention, on entend par lysat cellulaire un mélange d'antigènes intra-cellulaires et/ou membranaires, obtenu selon un protocole de lyse cellulaire bien connu de l'homme du métier, tel que notamment par lyse mécanique, chimique ou enzymatique de cellules.

Selon un mode préféré de l'invention, le médicament selon l'invention est destiné au traitement et/ou la prévention d'une infection d'origine bactérienne, virale, fongique, parasitaire ou provoquée par une levure, pour le traitement et/ou la prévention des cancers, c'est à dire contre toutes les maladies dues à une multiplication anormale des cellules, notamment les myélomes, lymphomes, leucémies, carcinomes du rein, du cerveau, de la prostate, du rectum, du colon, du pancréas, des ovaires, du poumon, du foie, du sein, les cancers cutanés choisi parmi les kératinomes et les carcinomes, les mélanomes.

Le médicament selon l'invention peut être notamment destiné à une administration par voie orale, par exemple sous la forme d'un comprimé, d'une gélule, d'une solution buvable, etc, par voie rectale, sous la forme d'un suppositoire, par voie parentérale, en particulier sous la forme d'une solution injectable, notamment par voie intraveineuse, intradermique, sous cutanée, par voie topique, sous la forme d'une crème, de pommade, lotions, selon des protocoles d'administration classiques bien connus de l'homme du métier. Le médicament selon l'invention peut comprendre en outre un agent de transport permettant de véhiculer au moins une fraction de lipoprotéines oxydées choisie parmi les VLDL oxydées et/ou les IDL oxydées et/ou LDL oxydées sous une forme permettant d'améliorer sa stabilité et/ou son activité immunostimulante et/ou sa capacité à induire une réponse immunitaire antitumorale. Cet agent de transport peut être notamment sous la forme d'une émulsion lipidique, d'une particule de type liposome, microsphère, nanosphère ou tout type de structure permettant l'encapsulation et la présentation sous forme particulaire de ladite fraction de lipoprotéines.

La posologie dépend de la gravité de l'affection et est ajustée pour obtenir un traitement thérapeutique adapté.

La présente invention concerne également un procédé pour la différenciation *in vitro* de cellules précurseur, notamment de monocytes, en cellules dendritriques matures selon lequel on dispose de cellules précurseur dans un milieu de culture approprié permettant leur différenciation et on additionne audit milieu, en une quantité prédéterminée, au moins une fraction de lipoprotéines oxydées choisies parmi les VLDL oxydées et/ou les IDL oxydées et/ou LDL oxydées. De préférence, les cellules précurseur sont des monocytes.

Par milieu approprié, on entend un milieu de culture comprenant tous les éléments nécessaires à la viabilité des cellules. On peut citer à titre d'exemple le milieu RPMI 1640 et ses dérivés. De préférence, le milieu de culture comprend des inducteurs de la différenciation des cellules précurseur en cellules dendritiques immatures. Préférentiellement, le milieu de culture comprend de l'interleukine IL-4 et le facteur GM-CSF (Granulocyte Macrophage-Stimulating factor).

Selon un mode préféré de l'invention, l'addition de lipoprotéines oxydées audit milieu est effectuée *in vitro,* en une quantité comprise notamment entre environ 1 et 20 µg/ml, préférentiellement entre environ 2,5 et 15 µg/ml et avantageusement entre environ 8 et 12 µg/ml. Selon un mode préféré de l'invention, la ou les fractions de lipoprotéines oxydées est ou sont ajoutée(s) dans ledit milieu entre le 1^{er} et 6^{e} jour de différenciation des cellules précurseur, préférentiellement entre le 4^{e} et le 5^{e} jour de différenciation des cellules précurseur. De préférence, les cellules précurseur sont des monocytes.

Selon un autre mode de réalisation de l'invention, le procédé comprend en outre l'addition d'un agent biologique dans le milieu de culture. De préférence, l'agent biologique est ajouté dans le milieu de culture à J5 ou J6.

L'invention concerne également l'utilisation desdites cellules dendritiques et d'au moins un agent biologique pour la fabrication d'un médicament destiné au traitement et/ou la prévention d'une infection d'origine bactérienne, virale, fongique, parasitaire ou provoquée par une levure, au traitement et/ou à la prévention des cancers. Ainsi, à titre non limitatif, on peut prélever un échantillon sanguin à un patient atteint d'un cancer ou d'une maladie infectieuse pour obtenir des monocytes. Ces monocytes sont ensuite différenciés en cellules dendritiques matures obtenues selon le procédé tel que défini précédemment, en présence d'un antigène antitumoral ou un antigène de l'agent infectieux contre lequel on veut accroître la réponse immunitaire du patient, qui sont alors utilisées pour la fabrication d'un médicament.

La présente invention concerne également un agent d'activation pour la différenciation de cellules précurseur, notamment de monocytes en cellules dendritiques matures, comprenant au moins une fraction de lipoprotéines oxydées choisies parmi les VLDL oxydées et/ou les IDL oxydées et/ou LDL oxydées. Par agent d'activation, on entend une molécule qui, dans une composition pharmaceutique, induit les effets d'une médication ou renforce ou complète les effets de la médication principale. Dans le cas d'une composition vaccinale, l'agent d'activation est un adjuvant qui est préférentiellement une molécule qui stimule la réponse immunitaire de l'organisme hôte.

L'invention concerne également une composition pharmaceutique comprenant une fraction de lipoprotéines oxydées choisies parmi les VLDL oxydées et/ou les IDL oxydées et/ou LDL oxydées et au moins un agent biologique en combinaison avec au moins un excipient pharmaceutiquement acceptable. L'excipient pharmaceutiquement acceptable est choisi selon la forme pharmaceutique et le mode d'administration souhaité et la composition pharmaceutique est obtenue selon les principes bien connus de l'homme du métier en galénique. La composition pharmaceutique selon l'invention est destiné notamment au traitement et/ou la prévention d'une infection d'origine bactérienne, virale, fongique, parasitaire ou provoquée par une levure, pour le traitement et/ou la prévention des cancers.

L'invention concerne enfin un procédé d'activation de lymphocytes T contre un agent biologique déterminé caractérisé en ce que :
- on dispose de cellules précurseur dans un milieu de culture approprié permettant leur différenciation ;
- on additionne audit milieu, en une quantité prédéterminée, au moins une fraction de lipoprotéines oxydées choisies parmi les VLDL oxydées \ et/ou les IDL oxydées et/ou LDL oxydées.
- on ajoute au moins un agent biologique dans le milieu de culture; et
- on cocultive les cellules dendritiques matures en présence de lymphocytes T.

De préférence, les cellules précurseur sont des monocytes. Selon un mode de réalisation préféré de l'invention, l'activation des lymphocytes T est réalisée *in vitro.*

Les exemples qui suivent sont données à titre explicatifs et n'ont aucun caractère limitatif Ils permettront de mieux comprendre l'invention.

### Exemple 1 - Différenciation de monocytes en culture en cellules dendritiques en absence de LDL oxydés dans le milieu de culture

*Isolement, mise en culture et initiation de la différenciation des monocytes -* Les monocytes sont isolés de sang périphérique humain par une première centrifugation suivant le gradient de densité (400g ; 20 minutes) dans du Ficoll-Hypaque, suivie d'une deuxième centrifugation (400g ; 20 minutes) dans une solution à 50% en Percoll. Les monocytes sont ensuite purifiés par déplétion immunomagnétique (Dynal, Oslo, Norway), en utilisant un cocktail d'anticorps monoclonaux anti CD 19 (hybridome 4G7) (Becton Dickinson, Francklin Lakes, NJ, USA), anti-CD3 (OKT3, American Type Culture Collection, Rockeville, MD) et anti-CD56 (NKH1, Beckman Coulter, Fullerton, CA, USA). Les monocytes ainsi obtenus sont alors purifiés à au moins 90%, comme montré par l'absence des marqueurs CD1a et CD14. La différenciation des monocytes en cellules dendritiques est initiée par 40ng/ml de GM-CSF (Granulocyte Macrophage-Colony Stimulating Factor) humain recombiné et 250U/ml d'interleukine IL-4 humain recombiné.

Les monocytes sont mis en culture dans le milieu RPMI 1640 (Life technologies, Rockeville, MD, USA) enrichi par 2mM de glutamine (Life technologies), 10mM d'Hepes (Life Technologies), 40ng/ml de gentamycine (Life technologies) et
- soit 10% de sérum de veau foetal (Biowest, Nuailles, France) (ou milieu de culture FCS)
- soit 10% de sérum de veau foetal dépourvu de lipoprotéines (Sigma, St Quentin-Fallavier, France) (ou milieu de culture LPDS). Ce milieu est choisi pour se placer dans des conditions optimales pour obtenir un effet des LDL oxydés.

*Phénotype des cellules en culture -* Le phénotype des cellules en culture 6 jours après l'initiation de la différenciation des monocytes est analysé par cytométrie de flux sur un FACSCalibur (Becton Dickinson, Francklin Lakes, NJ, USA) par l'utilisation de conjugué FITC (isothiocyanate de fluorosceine) anti-CD14, anti-HLA-DR, anti-CD80 et conjugué PE (phycoerythrine) anti-CD1a, anti-CD83, anti-CD86, anti-CD40 (Beckman Coulter). Il est connu de l'homme du métier que les monocytes ont préférentiellement un phénotype CD14+ CD1a-, les cellules dendritiques immatures ont préférentiellement un phénotype CD14- CD1a+ CD86-, les cellules dendritiques matures ont préférentiellement un phénotype CD14- CD1a intermédiaire- CD86+.

*Après 5 jours de culture en présence de GM-CSF et IL-4, plus de 95% des monocytes en culture présentent un phénotype comparable à celui de cellules dendritiques immatures. Les résultats sont comparables lors de la culture des monocytes dans un milieu FCS ou dans un milieu LPDS.*

### Exemple 2 - Différenciation de monocytes en culture en cellules dendritiques en présence de LDL oxydés dans le milieu de culture.

*Préparation des LDL -* Les LDL (low density protéine; densité comprise entre 1,025 et 1,055 g/ml) sont isolés de plasma humain par centrifugation. La densité du plasma a été amenée à 1,025g/ml par l'utilisation de NaBr. Après une première centrifugation réalisée à 100 000 tours par minute, à une température de 4°C pendant 4h (centrifugeuse Beckman TL 100), la fraction supérieure contenant les chylomicrons, les VLDL (very low density protein) et les IDL (intermediate density lipoprotein) est éliminée. La densité est alors ajustée à 1,055g/ml avec du NaBr. Une deuxième centrifugation est réalisée d'une façon comparable à la 1^{ère} centrifugation décrite précédemment. La fraction supérieure, contenant les LDL, est récupérée, dialysée contre une solution NaCl 150mM, EDTA 2,4mM, pH 7,2 à 4°C et filtrée à 0,45µm et conservée dans l'azote.

Le contenu en protéine de cette fraction de LDL est déterminé par la technique dite Coomassie Protein Micro-assay (nom commercial, Pierce, Rockford, IL, USA).

Le contenu en lipides de cette fraction de LDL est déterminé par l'utilisation des kits Cholesterol RTU, Triglycerides enzymatic PAP150 et Phospholipids enzymatic PAP 150 (noms commerciaux, bioMérieux, Marcy l'Etoile, France).

L'analyse montre que les LDL sont composés de 23 ± 1% de protéines (protéine Apo B uniquement), 41 ± 4% de cholesterol, 24 ± 1 % de phospholipides et 13 ± 4% de triglycérides. Les endotoxines présentes dans la fraction sont estimées par un test E-toxate (nom commercial, Sigma, St Quentin-Fallavier, France). La concentration en endotoxines est inférieure à 0,6 pg/ml dans la concentration finale de LDL ou de LDL oxydés.

*Oxydation des LDL -* La concentration en LDL est ajustée à 1mg/ml par dilution dans du PBS. L'EDTA est éliminée par dialyse contre du PBS à 4°C. L'oxydation par Cu²⁺ est effectuée à 37°C pendant 24h par dialyse contre une solution PBS, CuSO₄, 5µM. La réaction est stoppée par addition de Toluene Hydroxy-butyl (ref. B1378, Sigma, St Quentin-Fallavier, France), 40µM et dialyse poussée à 4°C contre du PBS contenant de l'acide diethylene diamine pentaacétique, 100µM. Le degré d'oxydation est déterminé par la production de malondialdehyde mesurée par la technique dite LPO-586 assay (Oxis, Portland, OR, USA).

*Isolement, mise en culture et initiation de la différenciation des monocytes en présence de LDL oxydés* - Ces étapes sont effectuées suivant le protocole de l'exemple 1. , en présence de LDL oxydés. Pour cela, 10µg/ml de LDL oxydés sont ajoutés dans le milieu de culture FCS ou LPDS à JO, jour où la culture des monocytes est initiée, J1, J2, J3, J4, J5 et J6, correspondant respectivement au 1^{er}, 2^{e}, 3^{e}, 4^{e}, 5^{e} et 6^{e} de culture.

*Phénotype des cellules en culture* - Le phénotype des cellules en culture est analysé, quant à la présence des marqueurs CD86 suivant le protocole de l'exemple 1.
- six jours après l'initiation de la différenciation des monocytes lorsque les LDL oxydés ont été ajoutés à J0, J3, J4, J5
- sept jours après l'initiation de la différenciation des monocytes lorsque les LDL oxydés ont été ajoutés à J6

Les cellules obtenues par différenciation des monocytes en FCS et en présence de LDL oxydés à partir de J0 expriment plus de marqueur CD86 que les cellules obtenues par différenciation des monocytes en l'absence de LDL oxydés. Les résultats concernant la présence du marqueur CD86 (exprimés en intensité moyenne de fluorescence) par des cellules en culture dans un milieu LPDS en absence (contrôle) ou en présence de 10µg/ml de LDL oxydés ajoutés à J0, J3, J4, J5 et J6 sont présentés dans le tableau 1.

**TABLEAU 1 - Expression du marqueur CD86 en l'absence ou en présence de LDL oxydés (10µg/ml ajoutés à J0, J3, J4, J5 ou J6**

| | **Contrôle** | **JO** | **J3** | **J4** | **J5** | **J6** |
|---|---|---|---|---|---|---|
| **CD86** | 44 | 187 | 178 | 370 | 403 | 173 |

L'induction de l'expression de CD86 est optimale au 4^{e} ou au 5^{e} jour de différenciation des monocytes et est dépendant de la dose de LDL oxydés ajoutée, comme représenté dans le tableau 2.

**TABLEAU 2 - Expression du marqueur CD86 en l'absence ou en présence de LDL oxydés à une concentration de 2,5 ; 5 ou 10µg/ml ajoutés à J5**

| | **Contrôle** | **2,5** | 5 | **10** |
|---|---|---|---|---|
| **CD86** | 100 | 195 | 336 | 528 |

De plus, en milieu LPDS, l'ajout de LDL oxydés à JO et, d'une façon plus marqué, à J5 dans le milieu de culture de monocytes en voie de différenciation induit un phénotype caractéristique de cellules dendritiques matures objectivé par les marqueurs CD83, CD80, CDS6, MHC de classe II, CD40 par comparaison aux monocytes en culture en l'absence de LDL oxydés (contrôle) (tableau 3).

**TABLEAU 3 - Expression des marqueur CD83, CD80 et CD86 en l'absence (contrôle) ou en présence de LDL oxydés (10µg/ml) ajoutés à J0 ou J5**

| | **CD83** | **CD80** | **CD86** |
|---|---|---|---|
| **Contrôle** | 3,3 | 7,5 | 44 |
| **J0** | 6,3 | 4,6 | 187 |
| **J5** | 20,6 | 16,2 | 403 |

*Après 6 jours de culture en présence de GM-CSF et IL-4 et de LDLox*, *ajoutés notamment entre le 4*^{*e*} *et le 5*^{*e*} *jour de différenciation*, *les cellules obtenues présentent un phénotype comparable à celui de cellules dendritiques matures*.

### Exemple 3 - Différenciation de monocytes en culture en cellules dendritiques en présence de LDL non oxydés et/ou de LDL oxydés dans le milieu de culture.

Le protocole utilisé dans cet exemple est comparable à celui présenté dans l'exemple 2.
Ainsi, les cellules présentées dans cet exemple sont obtenues après 6 jours de différenciation des monocytes en milieu LPDS réalisée
- en l'absence de LDL oxydés (selon le protocole de l'exemple 1),
- en présence de LDL non oxydés (50 µg/ml; préparation selon le protocole décrit dans l'exemple 2), ajouté dans le milieu de culture au 5^{e} jour de différenciation des monocytes
- en présence de LDL oxydés 10µg /ml ; préparation selon le protocole décrit dans l'exemple 2), ajouté dans le milieu de culture au 5^{e} jour de différenciation des monocytes
- en présence de LDL oxydés (10µg/ml) et de LDL non oxydés (50µg/ml), ajouté dans le milieu de culture au 5^{e} jour de différenciation des monocytes *Phénotype des cellules en culture* - Le phénotype des cellules en culture est analysé, quant à la présence du marqueur CD86 suivant le protocole de l'exemple 1, six jours après l'initiation de la différenciation des monocytes. Les résultats sont présentés dans le tableau 4.

**TABLEAU 4 - Expression du marqueur CD86 en l'absence ou en présence de LDL oxydés, de LDL non oxydés, de LDL oxydés et LDL non oxydés**

| | Contrôle | LDL ox | LDL | LDL+LDLox |
|---|---|---|---|---|
| CD86 CD86 | 53 | 439 | 79 | 232 |

Ainsi, les LDL non oxydés ajoutés dans le milieu de culture à J5 n'ont pas d'effet sur l'expression du marqueur CD86, suggérant que, contrairement aux LDL oxydés, les LDL non oxydés n'ont aucun effet sur la différenciation des monocytes en cellules dendritiques matures. De plus, un excès de LDL non oxydés dans le milieu de culture bloque l'action des LDL oxydés sur la différenciation des monocytes en cellules dendritiques matures.

*Après 6 jours de culture en présence de GM-CSF et IL-4 et de LDL non oxydés ou de LDL non oxydés en excès par rapport aux LDL oxydés, ajoutés notamment entre le 4*^{*e*} *et le 5*^{*e*} *jour de différenciation, les cellules obtenues présentent un phénotype proche de celui de cellules dendritiques immatures*.

### Exemple 4 - Capacité d'internalisation des cellules obtenues par différenciation de monocytes en l'absence ou en présence de LDL oxydés dans le milieu de culture

Les cellules sont obtenues après 6 jours de différenciation des monocytes en milieu LPDS réalisée
- en l'absence de LDL oxydés (selon le protocole de l'exemple 1)
- en présence de 10µg/ml de LDL oxydés ajoutés dans le milieu de culture au 5^{e} jour de différenciation des monocytes (J5, selon le protocole de l'exemple 2)

Au 6^{e} jour de culture, les cellules sont remises en suspension dans un milieu de culture FCS et incubées à 37°C :
- pendant 30 minutes avec 1mg/ml de FITC-T70-Dextran (Sigma) afin d'estimer la capacité d'internalisation de ces cellules par endocytose par récepteur
- pendant 30 minutes avec 1mg/ml de Lucifer Yellow (ref. L0259, Sigma, St Quentin-Fallavier, France) afin d'estimer la capacité d'internalisation de ces cellules par pinocytose
- pendant 3 heures avec carboxylate-modified yellow-green FluoSpheres (nom commercial, 0,45 µm, Molecular Probes, Leiden, The Netherlands) afin d'estimer la capacité d'internalisation de ces cellules par macropinocytose.

L'internalisation est stoppée sur glace par un tampon PBS froid contenant 1% de BSA (Bovine sérum Albumin, albumine sérique bovine) et 0,05% de NaN₃. Les cellules sont lavées 3 fois à 4°C dans ce même tampon et la fluorescence est quantifiée par FACScalibur (nom commercial, Becton Dickinson).

Comme le montre le tableau 5, les capacités d'internalisation par endocytose, pinocytose et macropinocytose sont fortement diminuées lors de l'ajout de 10µg/ml de LDL oxydés dans le milieu de culture à J5 par rapport au contrôle réalisé en l'absence de LDLoxydés.

**TABLEAU 5 - Capacité d'internalisation par endocytose, pinocytose et macropinocytose des monocytes différenciés en absence (contrôle) ou en présence de LDL oxydés (10µg/ml ajoutés à J5)**

| | **Endocytose** | **Pinocytose** | **Macropinocytose** |
|---|---|---|---|
| **Contrôle** | 100% | 100% | 100% |
| **10 µg/ml de LDL ox** | 41% | 39% | 54% |

La réduction des capacités d'internalisation est une des caractéristiques des cellules dendritiques matures. Notons que les cellules obtenues en présence de LDL non oxydés ou de LDL non oxydés en excès par rapport aux LDL oxydés, ne présentent pas cette réduction des capacités d'internalisation, suggérant que les cellules ainsi obtenues ne sont pas des cellules dendritiques matures.

Ces résultats montrent que en présence de LDL oxydés, ajoutés au 5^{*e*} jour de différenciation, les monocytes ont une forte tendance à se différencier en cellules présentant les caractéristiques fonctionnelles de cellules dendritiques matures.

### Exemple 5 - Quantification de la sécrétion de cytokines par les cellules obtenues par différenciation de monocytes en l'absence ou en présence de LDL oxydés

Les cellules sont obtenues après 6 jours de différenciation des monocytes en LPDS réalisée
- en l'absence de LDL oxydés (protocole de l'exemple 1),
- en présence de 10µg/ml de LDL oxydés ajoutés dans le milieu de culture à 5^{e} jour de différenciation des monocytes (J5, protocole de l'exemple 2).

La présence de cytokines, notamment les interleukines IL10 (cytokine anti inflammatoire) et IL12p70 (cytokine pro inflammatoire), sécrétées par les cellules dendritiques matures, est déterminée par un kit ELISA spécifique pour les cytokines (Endogen, Woburn, MA, USA). Les résultats sont présentés dans le tableau 6.

**TABLEAU 6- Sécrétion de cytokines par les monocytes différenciés en absence (contrôle) ou en présence de LDL oxydés (10µg/ml ajoutés à J5)**

| **Sécrétion de cytokines (pg/ml)** | **IL10** | **IL12p70** |
|---|---|---|
| **Contrôle** | 12 ± 1 | 9 ± 11 |
| **10 µg/ml de LDL ox** | 17 ± 8 | 159 ± 24 |

Les cellules obtenues par différenciation des monocytes en présence de LDL oxydés ajoutés dans le milieu de culture au 5^{e} jour de différenciation des monocytes ont acquis la capacité à sécréter l'interleukine IL12p70 par rapport aux cellules obtenues par différenciation des monocytes en l'absence de LDL oxydés (contrôle), qui est une des cytokines principalement sécrétée par des cellules dendritiques matures. La sécrétion d'IL10 n'est obtenue dans aucune des conditions.

*Ces résultats montrent une plus forte tendance des monocytes à se différencier directement en cellules présentant un phénotype et une fonctionnalité comparable à celui de cellules dendritiques matures lors de l'ajout de LDL oxydés au 5*^{*e*} *jour de différenciation.*

### Exemple 6 - Capacité des cellules obtenues par différenciation de monocytes en l'absence ou en présence de LDL oxydés à stimuler les lymphocytes T

Les cellules sont obtenues après 6 jours de différenciation des monocytes en milieu de culture LPDS réalisée
- en l'absence de LDL oxydés, dans un milieu de culture LPDS selon le protocole de l'exemple 1
- en présence de 10µg/ml de LDL oxydés ajoutés dans un milieu de culture LPDS au 5^{e} jour de différenciation des monocytes (J5, protocole de l'exemple 2)

Les lymphocytes T naïfs sont isolés de sang périphérique humain. Les cellules mononuclées du sang périphérique sont isolées par centrifugation (400g, 20 minutes) selon le gradient de densité en présence de Ficoll-Hypaque. Après élimination des monocytes sur gradient de Percoll, les lymphocytes du sang périphérique se situent dans la fraction dense. Les lymphocytes T sont purifiés par déplétion immunomagnétique par l'utilisation d'un cocktail d'anticorps monoclonaux anti-CD19 (anticorps 4G7), anti-CD16 (anticorps 3G8), anti-CD56 (anticorps NKH1), anti-glycophorin A (anticorps 11E4B7.6) et anti-CD14 (anticorps RMPO52), commercialisés par Beckman Coulter.

Les lymphocytes T purifiés, comme le montre la présence de marqueur CD3, sont cultivés dans des plaques de culture de 96 puits à fond plat avec les cellules présentatrices d'antigène. Par cellules présentatrices d'antigène on entend les monocytes différenciés en présence ou non de LDL oxydés. 2x10⁵ cellules T allogéniques ou syngéniques sont cultivées dans 200µl de milieu de culture selon un rapport de 1:5, 1:10 ou 1:20 cellules présentatrices d'antigène / cellules T. Après 4 jours, 50µl du surnageant de culture sont utilisés pour déterminer la sécrétion d'IL-2 (interleukine 2) par un kit ELISA (Endogen, Woburn, MA, USA), et remplacés par 50µl d'un milieu contenant 1µCi de [³H]-Thymidine. Après 16h de culture, les cellules sont collectées sur du papier filtre et l'incorporation de thymidine traduisant la prolifération des lymphocytes T, est mesurée par l'utilisation d'un compteur Matrix 9600 Direct beta (nom commercial, Packard, Meriden, CT, USA).

### a) Sécrétion d'interleukine 2 (IL-2)

Comme le présente le tableau 7, la sécrétion d'IL2 par les lymphocytes T allogéniques (exprimée classiquement selon le ratio cellules dendritiques/lymphocyte T, ratio DC/LT) est induite lorsque les cellules dendritiques proviennent de la différenciation de monocytes en présence de LDL oxydés ajoutés à J5, par comparaison aux résultats obtenus en l'absence de LDL oxydés (contrôle).

**TABLEAU 7 - Stimulation de la sécrétion d'IL2 par les lymphocytes T allogéniques (selon le ratio DC/LT) induite par les monocytes différenciés en absence (contrôle) ou en présence de LDL oxydés (10µg/ml ajoutés à J5)**

| | | | | |
|---|---|---|---|---|
| **Ratio DC/LT** | **0** | **0,05** | **0,1** | **0,2** |
| **Contrôle** | 0 | 72 | 64 | 84 |
| **10 µg/ml de LDL ox** | 0 | 189 | 373 | 454 |

Ces résultats montrent une augmentation des capacités d'allostimulation des cellules obtenus après différenciation des monocytes en présence de LDL oxydés.

### b) Prolifération de lymphocytes T

Comme le présente le tableau 8, la prolifération des lymphocytes T allogéniques, prolifération estimée par l'incorporation de ³H-thymidine induite par les cellules présentatrices d'antigène est fortement augmentée lorsque ces cellules sont obtenues par différenciation des monocytes en présence de LDL oxydés à J5, par comparaison aux résultats obtenus en l'absence de LDL oxydés (contrôle). Cet effet est dépendant de la dose de LDL oxydés présente dans le milieu de culture (2,5; 5 ou 10 µg/ml).

**TABLEAU 8 - Incorporation de ³H-thymidine (coup par minute) par les lymphocytes T allogéniques induite par des monocytes différenciés en absence (contrôle) ou en présence de LDL oxydés à une concentration de 2,5 ; 5 ou 10µg/ml ajoutés à J5**

| | **Contrôle** | **2,5** | **5** | **10** |
|---|---|---|---|---|
| ^{**3**}**H-thymidine** | 11712 ± 2476 | 13650 ± 1703 | 14906 ± 3387 | 25010 ± 1754 |

Les monocytes différenciés en présence de LDL oxydés (10µg/ml) à J5 sont également capables d'induire la prolifération de lymphocytes T syngéniques beaucoup plus efficacement que les monocytes différenciés en l'absence de LDL oxydés (contrôle), c'est à dire les cellules dendritiques immatures classiques comme présenté dans le tableau 9.

**TABLEAU 9 - Incorporation de ³H-thymidine (coup par minute) par les lymphocytes T syngéniques induite par des monocytes différenciés en absence (contrôle) ou en présence de LDL oxydés (10µg/ml) ajoutés à J5**

| | **Contrôle** | **10 µg/ml de LDL ox** |
|---|---|---|
| ^{**3**}**H-thymidine** | 920 ± 331 | 2983 ± 501 |

*Ces résultats indiquent que les LDL oxydés favorise la production de cellules capables de présenter des peptides provenant de la dégradation de protéines exogènes internalisées, qui présentent de ce fait des caractéristiques fonctionnelles de **cellules dendritiques matures*** Notons que cette capacité de présentation de peptides n'est pas observée lorsque les cellules sont obtenues à partir de monocytes cultivés en présence de LDL non oxydés ou de LDL non oxydés en excès par rapport aux LDL oxydés, tels qu'obtenues selon le protocole de l'exemple 2.

### Exemple 7 - Activation primaire des lymphocytes T spécifiques de la protéase du virus de l'hépatite C par des cellules dendritiques matures obtenues selon l'invention

Les cellules dendritiques utilisées dans cet exemple sont préparées selon le protocole de l'exemple 3, en présence de l'antigène NS3 de la protéase du virus de l'hépatite C. Après 6 jours de différenciation des monocytes en présence de LDL oxydés (10µg/ml) et/ou de LDL non oxydées (50µg/ml), ajoutés à J5 dans le milieu de culture, les cellules ainsi obtenues sont co-cultivées pendant 5 jours en présence de l'antigène NS3 de la protéase du virus de l'hépatite C et:
- de lympocytes T totaux, obtenus selon le protocole de l'exemple 5, ou
- de lymphocytes T CD 4+, obtenus selon le protocole de l'exemple 5, avec la variante consistant à rajouter dans le cocktail d'anticorps monoclonaux (Beckman Coulter) un anticorps anti-CD8 (Beckman Coulter) lors de la déplétion.

La sécrétion d'interféron gamma par les lymphocytes T totaux ou T CD4+, représentatif d'une réponse de type Th1, est alors mesurée (kit ELISA (Endogen, Woburn, MA, USA).

Les résultats obtenus sur les lymphocytes T totaux sont présentés dans le tableau 10 et les résultats obtenus sur les lymphocytes T CD4+ sont présentés dans le tableau 11.

**TABLEAU 10 - Sécrétion d'interféron gamma (pg/ml) par les lymphocytes T syngéniques spécifiques de l'antigène NS3 et activés par des cellules obtenues par différenciation des monocytes en présence de LDL oxydés, de LDL non oxydés ou de LDL oxydés + LDL non oxydés**

| **NS3 (µg/ml)** | **Contrôle** | **LDLox** | **LDL** | **LDLox + LDL** |
|---|---|---|---|---|
| 0 | 14 ± 6 | 24 ± 9 | 17 ± 5 | 44 ± 21 |
| 100 | 122 ± 39 | 723 ± 388 | 139 ± 113 | 112 ± 94 |

Ces résultats suggèrent que les lymphocytes totaux présents cocultivés en présence de cellules dendritiques matures obtenues par différenciation de monocytes en présence de LDL oxydés ont développé une capacité accrue à sécréter l'interféron gamma, définissant une réponse immunitaire de type Th1 dirigé contre l'antigène NS3 du virus de l'hépatite C.

**TABLEAU 11- Sécrétion d'interféron gamma (pg/ml) par les lymphocytes T CD4+ spécifiques de l'antigène NS3 et activés par des cellules obtenues par différenciation des monocytes en présence de LDL oxydés, de LDL non oxydés ou de LDL oxydés + LDL non oxydés**

| **NS3 (µg/ml)** | **Contrôle** | **LDLox** | **LDLox + LDL** |
|---|---|---|---|
| 0 | 3 ± 2 | 5 ± 4 | 4 ± 3 |
| 100 | 43 ± 40 | 809 ± 180 | 84 ± 50 |

Ces résultats suggèrent que les lymphocytes CD4+ cocultivés en présence de cellules dendritiques matures obtenues par différenciation de monocytes en présence de LDL oxydés ont développé une capacité accrue à sécréter l'interféron gamma, définissant une réponse immunitaire de type Th1 dirigé contre l'antigène NS3 du virus de l'hépatite C.

Ces résultats suggèrent que les cellules dendritiques obtenues en présence de LDL oxydés sont capables d'induire une réponse T primaire, propriété essentielle d'un adjuvant.

En conclusion, l'ajout de LDL oxydés dans le milieu de culture permet d'obtenir, à partir de monocytes des cellules dendritiques présentant les caractéristiques de cellules dendritiques matures notamment quant à leur capacité reduite d'endocytose et leur capacité à stimuler les lymphocytes T allogéniques et à activer des lymphocytes T syngéniques naïfs spécifiques d'un antigène. Ces résultats ne sont pas obtenus lors de l'ajout de LDL non oxydés dans le milieu de culture, et l'ajout de LDL non oxydés en excès dans le milieu de culture bloque les effets des LDL oxydés sur la différenciation des monocytes en cellules dendritiques matures. Notons toutefois que les cellules obtenues selon l'invention présentent également des caractéristiques qui leur sont propres, notamment quant à la sécretion de certaines cytokines.

## Revendications

1. Utilisation d'au moins une fraction de lipoprotéines oxydées choisies parmi les VLDL oxydées et/ou les IDL oxydées et/ou LDL oxydées pour obtenir la différenciation, in vitro, de cellules précurseur en cellules dendritiques matures.

2. Utilisation selon la revendication 1, **caractérisée en ce que** les cellules précurseur sont des monocytes.

3. Utilisation selon l'une quelconques des revendications 1 à 2, **caractérisée en ce que** la fraction de lipoprotéines oxydées consiste en une fraction de LDL oxydés.

4. Utilisation d'au moins une fraction de lipoprotéines oxydées choisies parmi les VLDL oxydées et/ou les IDL oxydées et/ou LDL oxydées pour la fabrication d'un médicament destiné à la différenciation de cellules précurseur en cellules dendritiques matures.

5. Utilisation selon la revendication 4 **caractérisée en ce que** les cellules précurseur sont des monocytes.

6. Utilisation selon l'une quelconque des revendications 4 à 5, **caractérisée en ce que** la fraction de lipoprotéines oxydées consiste en une fraction de LDL oxydés.

7. Utilisation selon l'une quelconque des revendication 4 ou 6 **caractérisé en ce qu'**elle comprend en outre l'utilisation d'au moins un agent biologique.

8. Utilisation selon la revendication 7, **caractérisée en ce que** l'agent biologique est choisi parmi les antigènes de bactéries, de virus, de levures, de parasites, de champignons, les antigènes tumoraux, les lysats de cellules tumorales autologues et/ou hétérologues.

9. Utilisation selon le revendication 7, **caractérisée en ce que** l'agent biologique est un acide nucléique qui code pour au moins un antigène choisi parmi les antigènes de bactéries, de virus, de levures, de parasites, de champignons, les antigènes tumoraux.

10. Utilisation selon l'une quelconque des revendications 7 à 9 pour la fabrication d'un médicament pour le traitement et/ou la prévention d'une infection d'origine bactérienne, virale, fongique, parasitaire ou provoquée par une levure.

11. Utilisation selon la revendication 7 à 9 pour la fabrication d'un médicament pour le traitement et/ou la prévention des cancers.

12. Procédé pour la différenciation in *vitro* de cellules précurseur en cellules dendritiques matures selon lequel
a) on dispose de cellules précurseur dans un milieu approprié permettant leur différenciation,
b) on additionne audit milieu, en une quantité prédéterminée, au moins une fraction de lipoprotéines oxydées choisies parmi les VLDL oxydées et/ou les IDL oxydées et/ou LDL oxydées.

13. Procédé selon la revendication 12 **caractérisé en ce que** les cellules précurseur sont des monocytes.

14. Procédé selon l'une quelconque des revendications 12 ou 13, selon lequel on additionne une fraction de LDL oxydées.

15. Procédé selon l'une quelconque des revendications 12 à 14, selon lequel les fractions de lipoprotéines oxydées ou la fraction de lipoprotéines oxydées sont ou est additionnée(s) audit milieu en une quantité comprise entre environ 1 à 20 µg/ml, préférentiellement entre environ 2,5 et 15 µg/ml et avantageusement entre 8 et 12 µg/ml.

16. Procédé selon l'une quelconque des revendications 12 à 14, selon lequel les fractions de lipoprotéines oxydées ou la fraction de lipoprotéines oxydées sont ou est ajoutée(s) dans ledit milieu entre le 3^{e} et 6^{e} jour de différenciation des cellules précurseur, préférentiellement entre le 4^{e} et le 5^{e} jour de différenciation des monocytes.

17. Procédé selon l'une quelconque des revendications 12 à 16 **caractérisé en ce qu'**on ajoute en outre un agent biologique dans le milieu de culture.

18. Composition pharmaceutique comprenant au moins une fraction de lipoprotéines oxydées choisies parmi les VLDL oxydées et/ou les IDL oxydées et/ou LDL oxydées comme agent d'activation.

19. Composition pharmaceutique selon la revendication 18, **caractérisée en ce que** la fraction de lipoprotéines oxydées consiste en une fraction de LDL, oxydées.

20. Composition pharmaceutique selon l'une des revendications 18-19 comprenant au moins un agent biologique en combinaison avec au moins un excipient pharmaceutiquement acceptable.

21. Procédé pour l'activation de lymphocytes T *in vitro* **caractérisé en ce que**:
c) on dispose de cellules précurseur dans un milieu approprié permettant leur différenciation,
d) on additionne audit milieu, en une quantité prédéterminée, au moins une fraction de lipoprotéines oxydées choisies parmi les VLDL oxydées et/ou les IDL oxydées et/ou LDL oxydées;
e) on ajoute au moins un agent biologique dans le milieu de culture et on cocultive les cellules dendritiques matures en présence de lymphocytes T.

## Patentansprüche

1. Verwendung von zumindest einer Fraktion von oxidierten Lipoproteinen, die ausgewählt sind aus den oxidierten VLDL und/oder den oxidierten IDL und/oder den oxidierten LDL, um *in vitro* die Differenzierung von vorläuferzellen in reife dendritische Zellen zu erwirken.

2. Verwendung nach Anspruch 1, **dadurch gekennzeichnet, dass** die Vorlauferzellen Monozyten sind.

3. Verwendung nach Anspruch 1 oder 2, **dadurch gekennzeichnet, dass** die Fraktion von oxidierten Lipoproteinen aus einer Fraktion von oxidierten LDL besteht.

4. Verwendung von zumindest einer Fraktion von oxidierten Lipoproteinen, die ausgewählt sind aus den oxidierten VLDL und/oder den oxidierten IDL und/oder den oxidierten LDL, zur Herstellung eines Arzneimittels, das zur Differenzierung von vorläuferzellen in reife dendritische Zellen vorgesehen ist.

5. Verwendung nach Anspruch 4, **dadurch gekennzeichnet, dass** die vorlauferzellen Monozyten sind.

6. Verwendung nach Anspruch 4 oder 5, **dadurch gekennzeichnet, dass** die Fraktion von oxidierten Lipoproteinen aus einer Fraktion von oxidierten LDL besteht.

7. Verwendung nach Anspruch 4 oder 6, **dadurch gekennzeichnet, dass** diese zusätzlich die Verwendung von zumindest einem biologischen Agens umfasst.

8. Verwendung nach Anspruch 7, **dadurch gekennzeichnet, dass** dass biologische Agens ausgewählt ist aus den Antigenen von Bakterien, von Viren, von Hefen, von Parasiten, von Pilzen, aus tumorösen Antigenen, aus Lysaten von autologen und/oder heterologen tumorösen Zellen.

9. Verwendung nach Anspruch 7, **dadurch gekennzeichnet, dass** das biologische Agens eine Nukleinsäure ist, die für zumindest ein Antigen kodiert, das ausgewählt ist aus den Antigenen von Bakterien, von Viren, von Hefen, von Parasiten, von Pilzen, aus tumorösen Antigenen.

10. Verwendung nach einem der Ansprüche 7 bis 9 zur Herstellung eines Arzneimittels zur Behandlung und/oder Prävention einer Infektion mit bakteriellem, viralem, pilzartigem Ursprung, oder einer solchen, die durch eine Hefe hervorgerufen wurde.

11. Verwendung nach einem der Ansprüche 7 bis 9 zur Herstellung eines Arzneimittels zur Behandlung und/oder Prävention von Krebs.

12. Verfahren, um *in vitro* vorläuferzellen in reife dendritisehe Zellen zu differenzieren, nach dem
a) vorläuferzellen in ein geeignetes Medium gegeben werden, das deren Differenzierung ermöglicht,
b) zu dem Medium in einer bestimmten Menge zumindest eine Fraktion von oxidierten Lipoproteinen hinzugegeben wird, die ausgewählt sind aus den oxidierten VLDL und/oder den oxidierten IDL und/oder oxidierten LDL.

13. Verfahren nach Anspruch 12, **dadurch gekennzeichnet**, daas die Vorlaüferzellen Monozyten sind.

14. Verfahren nach Anspruch 12 oder 13, nach dem eine Fraktion von oxidierten LDL hinzugegeben wird.

15. Verfahren nach einem der Ansprüche 12 bis 14, nach dem die Fraktionen von oxidierten Lipoproteinen oder die Fraktion von oxidierten Lipoproteinen zu dem Medium in einer Menge, die bei etwa 1 bis 20 µg/ml, vorzugsweise bei etwa 2,5 bis 15 µg/ml und vorteilhafterweise bei 8 bis 12 µg/ml liegt, hinzugegeben werden bzw. hinzugegeben wird.

16. Verfahren nach einem der Ansprüche 12 bis 14, nach dem die Fraktionen von oxidierten Lipoproteinen oder die Fraktion von oxidierten Lipoproteinen in das Medium zwischen dem dritten und dem sechsten Tag der Differenzierung der Vorläuferzellen, vorzugsweise zwischen dem vierten und dem fünften Tag der Differenzierung der Monozyten, hinzugegeben werden bzw. hinzugegeben wird.

17. Verfahren nach einem der Ansprüche 12 bis 16, **dadurch gekennzeichnet, dass** zusätzlich ein biologisches Agens in das Kultivierungsmedium gegeben wird.

18. Pharmazeutische Zusammensetzung, die zumindest eine Fraktion von oxidierten Lipoproteinen, die ausgewählt sind aus den oxidierten VLDL und/oder den oxidierten IDL und/oder oxidierten LDL, als Aktivierungsagens aufweist.

19. Pharmazeutische Zusammensetzung nach Anspruch 18, **dadurch gekennzeichnet, dass** die Fraktion von oxidierten Lipoproteinen aus einer Fraktion von oxidierten LDL besteht.

20. Pharmazeutische Zusammensetzung nach Anspruch 18 oder 19, die zumindest ein biologisches Agens in Kombination mit zumindest einem pharmazeutisch akzeptablen Exzipienten aufweist.

21. Verfahren, um in *vitro* T-Lymphozyten zu aktivieren, **dadurch gekennzeichnet, dass**:
c) Vorläuferzellen in ein geeignetes Medium gegeben werden, das deren Differenzierung ermöglicht,
d) zu dem Medium eine bestimmte Menge von zumindest einer Fraktion von oxidierten Lipoproteinen hinzugegeben wird, die ausgewählt sind aus den oxidierten VLDL und/oder den oxidierten IDL und/oder oxidierten LDL;
e) zumindest ein biologisches Agens in das Kultivierungsmedium gegeben wird, und die reifen dendritischen Zellen in Gegenwart von T-Lymphozyten cokultiviert werden.

## Claims

1. Use of at least one fraction of oxidized lipoproteins chosen from oxidized VLDLs and/or oxidized IDLs and/or oxidized LDLs, for the differentiation, in vitro, of precursor cells into mature dendritic cells.

2. Use as claimed in claim 1, **characterized in that** the precursor cells are monocytes.

3. Use as claimed in either one of claims 1 to 2, **characterized in that** the fraction of oxidized lipoproteins consists of a fraction of oxidized LDLs.

4. Use of at least one fraction of oxidized lipoproteins chosen from oxidized VLDLs and/or oxidized IDLs and/or oxidized LDLs, for producing a medicinal product intended for the differentiation of precursor cells into mature dendritic cells.

5. Use as claimed in claim 4, **characterized in that** the precursor cells are monocytes.

6. Use as claimed in either one of claims 4 to 5, **characterized in that** the fraction of oxidized lipoproteins consists of a fraction of oxidized LDLs.

7. Use as claimed in either one of claims 4 or 6, **characterized in that** it also comprises the use of at least one biological agent.

8. Use as claimed in claim 7, **characterized in that** the biological agent is chosen from bacterial, viral, yeast, parasite or fungal antigens, tumor antigens, and autologous and/or heterologous tumor cell lysates.

9. Use as claimed in claim 7, **characterized in that** the biological agent is a nucleic acid which encodes at least one antigen chosen from bacterial, viral, yeast, parasite or fungal antigens, and tumor antigens.

10. Use as claimed in any one of claims 7 to 9, for producing a medicinal product for the treatment and/or prevention of an infection of bacterial, viral, fungal or parasitic origin or an infection caused by a yeast.

11. Use as claimed in claims 7 to 9, for producing a medicinal product for the treatment and/or prevention of cancers.

12. Method for the in *vitro* differentiation of precursor cells into mature dendritic cells, according to which
a) precursor cells are provided in a suitable medium which allows differentiation thereof,
b) a predetermined amount of at least one fraction of oxidized lipoproteins chosen from oxidized VLDLs and/or oxidized IDLs and/or oxidized LDLs is added to said medium.

13. Method as claimed in claim 12, **characterized in that** the precursor cells are monocytes.

14. Method as claimed in either one of claims 12 or 13, according to which a fraction of oxidized LDLs is added.

15. Method as claimed in any one of claims 12 to 14, according to which the fraction(s) of oxidized lipoproteins is or are added to said medium in an amount of between approximately 1 and 20 µg/ml, preferably between approximately 2.5 and 15 µg/ml, and advantageously between 8 and 12 µg/ml.

16. Method as claimed in any one of claims 12 to 14, according to which the fraction(s) of oxidized lipoproteins is or are added to said medium between the 3rd and 6th day of differentiation of the precursor cells, preferably between the 4th and the 5th day of monocyte differentiation.

17. Method as claimed in any one of claims 12 to 16, **characterized in that** a biological agent is also added to the culture medium.

18. Pharmaceutical composition comprising at least one fraction of oxidized lipoproteins chosen from oxidized VLDLs and/or oxidized IDLs and/or oxidized LDLs.

19. Pharmaceutical composition as claimed in claim 18, **characterized in that** the fraction of oxidized lipoproteins consists of fraction of oxidized LDLs.

20. Pharmaceutical composition as claimed in any one of claims 18-19, comprising at least one biological agent in combination with at least one pharmaceutically acceptable excipient.

21. A method for activating T lymphocytes *in vitro*, **characterized in that**:
c) precursor cells are provided in a suitable medium which allows differentiation thereof;
d) a predetermined amount of at least one fraction of oxidized lipoproteins chosen from oxidized VLDLs and/or oxidized IDLs and/or oxidized LDLs is added to said medium;
e) at least one biological agent is added to the culture medium and the mature dendritic cells are cocultured in the presence of T lymphocytes.
